# EUROPEAN PATENT APPLICATION

(11) **EP 2 700 419 A1**
(43) Date of publication of application: **26.02.2014**
(21) Application number: 12774056.1
(22) Date of filing: 02.03.2012
(51) Int. Cl.: A61L 15/28, A61F 13/02

(54) **WOUND DRESSING WITH BACTERIOSTASIS AND HYGROSCOPICITY**

(30) Priority: 20.04.2011 CN 201110099214
(71) Applicant: Foshan United Medical Technologies Ltd, Guangdong 528225 (CN)
(72) Inventor: WANG, Xiaodong, Foshan Guangdong 528225 (CN); ZHANG, Dawei, Foshan Guangdong 528225 (CN); MO, Xiaohui, Foshan Guangdong 528000 (CN); LV, Qiulan, Foshan Guangdong 528225 (CN); WU, Zhenjun, Changsha Hunan 410081 (CN)
(74) Representative: Hellmich, Wolfgang
(86) International application number: PCT/CN2012/071870
(87) International publication number: WO 2012/142879

(57) **Abstract**

A wound dressing with bacteriostatic and hygroscopicity, preparation method therefore, and the use thereof in preparing a product for treating chronic wounds. The dressing comprises chitosan fiber and modified cellulose fiber.

## Description

### FIELD OF THE INVENTION

The present invention involves a wound dressing comprising chemically modified chitosan fiber or unmodified chitosan fiber, and chemically modified cellulose fiber, providing clinical benefits in bacteriostatic and fluid absorption. The wound dressing is useful in treatment of chronic wounds, such as venous stasis ulcers, pressure ulcers, diabetic foot ulcers and other chronic ulcers.

### BACKGROUND OF THE INVENTION

It is well known that nurses are facing some challenges when selecting the right wound dressing for the management of chronic wounds. In addition to managing the wound exudates, they also need to consider providing a good healing environment for the wound. This good healing environment includes inhibiting the growth of microorganisms.

Chitosan has a bacteriostatic property by the existence of the amino groups in the molecule of chitosan. The positive charge of a chitosan molecule neutralizes the negative charge of cell membrane of bacteria inhibiting the growth of the bacteria. On the other hand, small molecules of chitosan penetrate through the bacterial cell membrane into the cell nucleus to inhibit enzyme formation. This can be observed in the general inhibition test. When the chitosan wound dressing is placed onto a Petri-dish for 24 hrs that has been covered with bacteria, a clear or less cloudy can be seen underneath the wound dressing whilst all other areas of the bottom of the Petri-dish displayed a cloudy appearance. This means that the area underneath the chitosan wound dressing has less bacterial growth than other areas which has not been covered by the chitosan dressing. This demonstrates that chitosan has a bacteriostatic property, which can be used to treat infected wounds.

Generally speaking, silver wound dressings also have bacteriostatic properties. However, a wound dressing containing silver which may be cytotoxic.

EP0690344 and US3589364 disclose an absorptive wound dressing manufactured by carboxymethyl cellulose fibers. WO2010/061225 discloses a method of preparing modified cellulose fiber by forming a water insoluble alkyl sulfonate cellulose fiber to improve its absorbency. According to the above patents, high absorbency can be obtained when the cellulose fibers are chemically modified. However this type of dressing does not have bacteriostatic properties.

Alginate dressings are also used in the management of chronic wounds. However alginates do not have bacteriostatic properties, its absorbency is also lower than that of chemically modified cellulose fibers.

EP0740554 and US6471982 disclose a fibrous wound dressing prepared by blending a gelling fiber such as an alginate fiber and a non-gelling fiber such as cellulose fiber. This blending method may reduce the cost of the product.

US7385101 discloses a wound dressing composed of a silver nylon fiber and an absorptive fiber. This dressing is used in managing the infected wounds.

US5836970 describes a wound dressing composed of chitosan fiber and alginate fiber. This patent disclosed the bacteriostatic and haemostatic properties of chitosan fiber and the absorbency of alginate fiber.

US6458460, EP0927013 and CN1303355 describe a wound dressing containing two gelling fibers, one is carboxymethyl cellulose fiber and the other one is alginate fiber. The mixture of these two fibers helps to improve the dressing absorbency, but does not have bacteriostatic property.

EP1318842 disclosed a wound dressing composing a silver fiber and a non-silver fiber. This wound dressing possesses antibacterial function as well as absorbency. This type of dressing generally is cytotoxic.

CN1313416 disclosed a method of blending cotton fibers and chitosan fibers. Although the purpose of this invention was not for wound care, it also disclosed that the product prepared by this method has antibacterial function.

Therefore it become a clinical need to develop a wound dressing that is very absorbent to wound fluid but can also provide some bacteriostatic functions.

### SUMMARY OF THE INVENTION

The present invention involves a wound dressing characterized in that the wound dressing comprises of chitosan fibers and chemically modified cellulose fibers. The chitosan fiber can be chemically modified or unmodified fiber. Particularly, the invention involves a wound dressing prepared by blending chitosan fibers and chemically modified cellulose fibers to obtain a fabric. Because of bacteriostatic property of the chitosan fiber and the high absorption capacity of the chemically modified cellulose, the dressing of the present invention can provide an ideal healing environment of bacteriostatic properties and fluid absorption to the chronic wound. Furthermore, the ratio of the chitosan fiber to chemically modified cellulose can be adjusted to suit the need of each type of the wound. For example for a wound that has a large amount of fluid but has not yet developed the wound infection, a dressing containing a small percentage of chitosan fiber is more suitable, as the small amount of chitosan fiber may be sufficient to prevent the wound from infection whilst the majority of the dressing is made of chemically modified cellulose which can provide the fluid absorption capacity needed for this type of the wound exudate. For example 30% of chitosan fiber and 70% of chemically modified cellulose fiber. However for the wound type that has already got infection, the dressing with more chitosan fiber is best suited, such as 70% of chitosan fiber and 30% of chemically modified cellulose fiber.

The present invention also relates to the application of the said wound dressing in the management of chronic wounds

Particularly, the invention involves a wound dressing wherein the wound dressing is composed of 5-95% w/w of chitosan fibers, 95-5% w/w chemically modified cellulose fibers, preferably 10-90% w/w of chitosan fibers, 90-10% w/w chemically modified cellulose fibers. All fiber percentage is based on the total weight of chitosan fiber and the chemically modified cellulose fibers. The wound dressing in the present invention is manufactured by blending of 5-95% w/w of chitosan fibers and 95-5% w/w chemically modified cellulose fibers. The blending is achieved during the carding and nonwoven process, i.e. the two fibers are weighed separately and blended together during or before the fiber opening stage, then carded together to form a nonwoven fabric made of a homogeneous mix of two fibers.

This invention also involves a method of manufacturing the said wound dressing by blending of chemically modified or unmodified chitosan fibers and chemically modified cellulose fibers through a nonwoven process, followed by slitting, cutting, packaging and sterilisation.

The wound dressing in the present invention can be used in the management of chronic wounds, such as venous stasis ulcers, pressure ulcers, diabetic foot ulcers and other chronic ulcers.

### BRIEF DESCRIPTION OF THE DRAWINGS

Fig. 1 shows the photo of the area underneath of the dressing prepared by blending 5% of chitosan fiber and 95% of chemically modified cellulose fiber at 1 day against *Staphylococcus aureus;*
Fig. 2 shows the photo of the area underneath of the dressing prepared by blending 95% of acylated chitosan fiber and 5% of chemically modified cellulose fiber at 1 day against *Escherichia coli;*
Fig. 3 shows the photo of the area underneath of the dressing prepared by blending 50% of acylated chitosan fiber and 50% of chemically modified cellulose fiber at 1 day against *Staphylococcus aureus;* and
Fig. 4 shows the device that was used in the wet strength tensile testing.

### DETAILED DESCRIPTION OF THE PREFERRED EMBODIMENTS

In an example of the present invention, chitosan fibers and chemically modified cellulose fibers were prepared respectively by their manufacturing process. Chitosan fiber can be prepared by first dissolving the chitosan powder in an acetic acid aqueous solution, then extruding the mixed chitosan solution (dope) into sodium hydroxide solution for precipitation. This is followed by washing, stretching, drying, cutting into staple fiber. The degree of deacetylation of the chitosan fiber is 50% or above, preferably 70% or above. Chemically modified chitosan fiber is prepared by reacting the chitosan fiber with certain chemicals so that the chitosan fiber becomes more absorbent or gelling. Typical examples of this kind of treatment are carboxymethylation or acylation treatments.

The chemically modified chitosan fiber used in the present invention is acylated chitosan fiber or carboxymethyl chitosan fiber. Carboxymethyl chitosan fiber is prepared by reacting the chitosan fiber with halogenated acetic acid. The acylated chitosan fiber is obtained by reacting the chitosan fiber with succinic anhydride. These chemical treatments make the chitosan fiber absorbent and gelling.

The chemically modified cellulose fiber used in the present invention is carboxymethyl cellulose fiber, preferably the carboxymethylated solvent spun cellulose fiber. Or the chemically modified cellulose fiber used in the present invention is a water insoluble cellulose alkyl sulfonate fiber. The chemically modified cellulose fiber is prepared by reacting standard cellulose fiber or solvent spun cellulose fiber (Lyocell) with certain chemicals for increased absorption capacity or gelling property. The preferred chemically modified cellulose fiber is carboxymethyl cellulose fiber or water insoluble cellulose alkyl sulfonate fiber.

The unmodified chitosan fiber has generally an absorbency to Solution A (a solution containing 8.298g sodium chloride and 0.368g calcium chloride dihydrate per liter) of 100% or 200%. The chemically modified chitosan fiber or cellulose fiber has an absorbency to Solution A (a solution containing 8.298g sodium chloride and 0.368g calcium chloride dihydrate per liter) of 500% or above, sometimes can be as high as 3000%.

It is well known that the strength of a wound dressing or a nonwoven material is different in the machine direction compared to the cross machine direction. The machine direction (MD) is the direction that materials move during the manufacturing. The cross machine direction (CD) is the direction 90 degree to the MD. Normally the strength of a nonwoven material in machine direction is lower than that in cross machine direction. Although it is difficult to distinguish machine direction and cross machine direction when the material is cut into square or rectangle dressing, the direction with lower strength can be considered as the MD. Therefore a wound dressing generally has two strengths, one is MD strength and the other is CD strength. The average strength (including average wet strength) is the average of MD and CD strengths. The average wet strength of the wound dressing in the present invention is 0.3 N/cm or above, preferably 0.5 N/cm, more preferably 1.0 N/cm, most preferably 1.8 N/cm or as high as 6.0 N/cm.

In an example of the present invention, chitosan fibers and chemically modified cellulose fibers are blended before or during the carding process. Usually, the blending process takes place in the fiber opening stage, followed by carding and needling processes. The carding process can further open and blend the two fibers to achieve a homogeneous mix. The typical nonwoven method is needle punching process.

In another example of the present invention, the chitosan fibers and the chemically modified cellulose fibers may contain surfactant, lubricant or antimicrobial agent. Some of these are process aids, others are for special purposes. For example, to apply Tween 20 onto the fiber surface can improve the process efficiency of the carding and the nonwoven process. For the purpose of enhancing the dressing's ability to kill bacteria and fungi, it will be necessary to add some antimicrobial agents to the fiber such as silver or PHMB.

In an example of the present invention, the content of chitosan fibers and the chemically modified cellulose fibers can be varied to suit the functional requirement of the wound dressing. The ratio of chitosan fiber can be between 5-95% w/w, calculated on the total weight of both fibers. The ratio of chemically modified cellulose fiber can be 95-5% w/w, calculated on the total weight of both fibers.

Preferably, the present invention involves a wound dressing composed of 10-90% w/w of chitosan fiber and 90-10% w/w of chemically modified cellulose fiber.

In the present invention, the fiber's linear density and length are controlled to suit the wound dressing manufacturing process. The linear density of the chitosan fibers and the chemically modified cellulose fibers is between 0.5 dtex to 5 dtex, preferably 2 dtex to 4 dtex. The length of the chitosan fibers and the chemically modified cellulose fibers is between 10mm to 125mm.

The present invention also involves a method of preparing the said wound dressing. The method comprises blending the chitosan fibers and the chemically modified cellulose fibers together during or before the fiber opening stage, then converting the blended fibers into a fabric through a nonwoven process, then cutting, packing and sterilizing. Preferably, the nonwoven process is needle punch process. Other nonwoven process can also be used. For example, one of the said fibers can be converted into a fabric first, and then the other fiber is laminated onto this pre-made fabric by needling or chemical bonding. Although the dressing manufactured by this method is not a homogeneous blend of the two fibers (chitosan fiber and chemically modified cellulose fiber), the dressing can still provide a bacteriostatic environment and high absorbency functions. Another method is to prepare a fabric that contains 100% chitosan fiber (either chemically modified or unmodified, or both) and a fabric that contains 100% chemically modified cellulose fiber first, and then laminate the two fabrics together by needling or chemical bonding.

According to the shape of different wounds, the wound dressing composed of chitosan fibers and chemically modified cellulose fibers can be cut into a square or rectangular shape to satisfy various applications in wound care.

The wound dressing in present invention is usually be packed by a known packaging material such as paper/poly, paper/paper, or foil/foil, and then sterilized by gamma irradiation or ETO.

The present invention can be further illustrated by the following examples.

### Example 1

Raw material: Chitosan fiber: linear density 2.0 dtex, fiber length 50 mm. The fiber contains 1% by weight of surfactant (Tween 20). The fiber's absorbency to the solution containing 8.298g/l of sodium chloride and 0.368 g/I of calcium chloride dehydrate (solution A) is over 110%. Chemically modified cellulose fiber: linear density 1.4 dtex, fiber length 38 mm. The fiber is modified by carboxymethylation reaction. The fiber's absorbency of a solution containing 8.298g/l of sodium chloride and 0.368 g/I of calcium chloride dehydrate (solution A) is 2200%.

100 g of chitosan fiber and 900 g of chemically modified cellulose fiber are blended and opened manually for 5 mins, then fed into a single cylinder card (Cuarnicard). The fibers are further blended into the hopper and the card, then opened and formed into a web. The web is crosslapped and needled into a nonwoven with a base weight of 130 gsm.

Cut the fabric into 10x10 cm, pack the dressing into pouches then sterilise the dressing by gamma irradiation.

The dressing has an absorbency of 18.5 g/g, a wet strength in CD direction of 0.45 N/cm, in MD direction of 0.17 N/cm, average 0.3 N/cm.

### Example 2

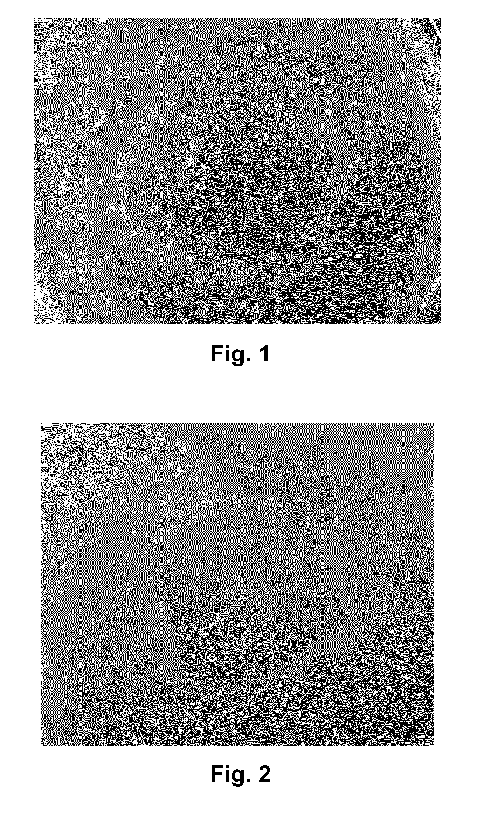

In order to observe the bacteriostatic performance of the wound dressing in Example 1, approximately 0.25 mL of *Staphylococcus aureus* at a concentration of 10E6 - 10E7cfu/mL was evenly coated on a Petri dish. Then the dressing obtained from Example 1 was cut into 2x2 cm and placed into the Petri dish. The Petri dish was then cultured at temperature of 37 °C, and observed for the growth of bacteria on the plate. Fig. 1 shows the area underneath the dressing at 1 days.

From Fig 1, it can be seen that the area underneath of the dressing is less cloudy than the rest of the Petri dish, indicating less growth of bacteria underneath of the dressing.

### Example 3

Raw material: Acylated Chitosan fiber: linear density 2.0 dtex, fiber length 50 mm. The fiber contains 1% by weight of surfactant (Tween 20). The fiber's absorbency to the solution containing 8.298g/l of sodium chloride and 0.368 g/I of calcium chloride dehydrate (solution A) is over 1500%. Chemically modified cellulose fiber: linear density 1.7 dtex, fiber length 50 mm. The fiber is modified by carboxymethylation reaction. The fiber's absorbency of the solution containing 8.298g/l of sodium chloride and 0.368 g/I of calcium chloride dehydrate (solution A) is 1500%.

Take 400 g of chitosan fiber and submerse the fiber in ethanol solution for 30 minutes. Squeeze the fiber to dry then place the fiber into a 0.1g/ml succinic anhydride solution (894g of succinic anhydride in 8940 ml of ethanol). Heat to 70 °C for 40 mins. Squeeze the fiber to dry then wash the fiber in an ethanol solution, followed by submersing the fiber in an ethanol solution containing Tween 20. The final steps are drying the fiber to an acceptable moisture content and cutting the fiber to a staple length.

Take 190 g of the above acylated chitosan and 10g of chemically modified cellulose fiber, blend and open two fibers manually for 5 mins, then fed the blend into a single cylinder card (Cuarnicard). The fibers are further blended into the hopper and the card, then opened and formed into a web. The web is crosslapped and needled punched into a nonwoven with a base weight of 130 gsm.

Cut the fabric into 10x10 cm, pack the dressing into pouches then sterilise the dressing by EtO.

The dressing has an absorbency of 14.6 g/g, a wet strength in CD direction of 2.5 N/cm, in MD direction of 1.1 N/cm, average 1.8 N/cm.

### Example 4

In order to observe the bacteriostatic performance of the wound dressing in Example 3, approximately 0.25 mL of *E. Coli* at a concentration of 10E6 - 10E7cfu/mL was evenly coated on a Petri dish. Then the dressing obtained from Example 3 was cut into 2x2 cm and placed into the Petri dish. The Petri dish was then cultured at temperature of 37 °C, and observed for the growth of bacteria on the plate. Fig. 2 shows the area underneath the dressing at 1 days.

From the Fig 2, it can be seen that the area underneath of the dressing is less cloudy than the rest of the Petri dish, indicating less growth of bacteria underneath of the dressing.

### Example 5

Raw material: Acylated Chitosan fiber: linear density 2.2 dtex, fiber length 75 mm. The fiber's absorbency of the solution containing 8.298g/l of sodium chloride and 0.368 g/I of calcium chloride dehydrate (solution A) is over 1500%. Chemically modified cellulose fiber: linear density 1.7 dtex, fiber length 50 mm. The fiber is modified by carboxymethylation reaction. The fiber's absorbency of the solution containing 8.298g/l of sodium chloride and 0.368 g/I of calcium chloride dehydrate (solution A) is 1500%.

Take 1000 g of the acylated chitosan and 1000 g of carboxymethyl cellulose fiber, blend and open two fibers manually for 5 mins, then fed the blend into a single cylinder card (Cuarnicard). The fibers are further blended into the hopper and the card, then opened and formed into a web. The web is crosslapped and needled punched into a nonwoven with a base weight of 110 gsm.

Cut the fabric into 10x10 cm, pack the dressing into pouches then sterilise the dressing by EtO.

The dressing has an absorbency of 14.6 g/g, a wet strength in CD direction of 1.5 N/cm, in MD direction of 0.5 N/cm, average 1.0 N/cm.

### Example 6

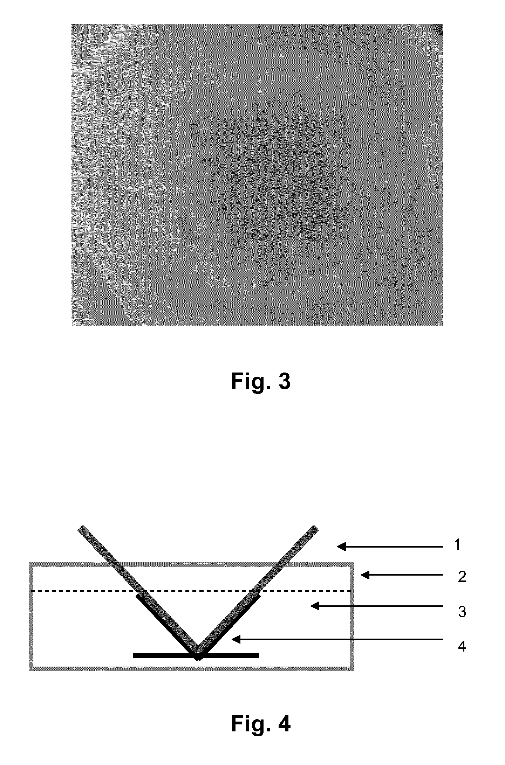

In order to observe the bacteriostatic performance of the wound dressing in Example 5, approximately 0.25 mL of *Staphylococcus aureus* at a concentration of 10E6 - 10E7cfu/mL was evenly coated on a Petri dish. Then the dressing obtained from Example 5 was cut into 2x2 cm and placed into the Petri dish. The Petri dish was then cultured at temperature of 37 °C, and observed for the growth of bacteria on the plate. Fig. 3 shows the area underneath the dressing at 1 days.

From the Fig 3, it can be seen that the area underneath of the dressing is less cloudy than the rest of the Petri dish, indicating less growth of bacteria underneath of the dressing.

### Example 7

Raw material: Acylated Chitosan fiber: linear density 2.2 dtex, fiber length 75 mm. The fiber's absorbency of the solution containing 8.298g/l of sodium chloride and 0.368 g/I of calcium chloride dehydrate (solution A) is over 800%. Chemically modified solvent spun cellulose fiber: linear density 1.4 dtex, fiber length 60 mm. The fiber is modified by carboxymethylation reaction. The fiber's absorbency of the solution containing 8.298g/l of sodium chloride and 0.368 g/I of calcium chloride dehydrate (solution A) is 3000%.

Take 1000 g of the acylated chitosan and 1000 g of carboxymethyl cellulose fiber, blend and open two fibers manually for 5 mins, then fed the blend into a single cylinder card (Cuarnicard). The fibers are further blended into the hopper and the card, then opened and formed into a web. The web is crosslapped and needled punched into a nonwoven with a base weight of 160 gsm.

Cut the fabric into 10x10 cm, pack the dressing into pouches then sterilise the dressing by EtO.

The dressing has an absorbency of 17.5 g/g, a wet strength in CD direction of 0.9 N/cm, in MD direction of 0.2 N/cm, average 0.55 N/cm.

### Example 8

Raw material: Acylated Chitosan fiber: linear density 2.2 dtex, fiber length 50 mm. The fiber's absorbency of the solution containing 8.298g/l of sodium chloride and 0.368 g/I of calcium chloride dehydrate (solution A) is over 800%. The fiber contains 1% of Tween 20. Carboxymethyl cellulose fiber: linear density 2.2 dtex, fiber length 50 mm. The fiber's surface was sprayed with about 3000 ppm PHMB as an antimicrobial fiber.

Take 400 g of the acylated chitosan and 100 g of above antimicrobial carboxymethyl cellulose fiber, blend and open two fibers manually for 5 mins, then fed the blend into a single cylinder card (Cuarnicard). The fibers are further blended into the hopper and the card, then opened and formed into a web. The web is crosslapped and needled punched into a nonwoven with a base weight of 100 gsm.

Cut the fabric into 10x10 cm, pack the dressing into pouches then sterilise the dressing by EtO.

The dressing has an absorbency of 12 g/g, a wet strength in CD direction of 0.8 N/cm, in MD direction of 0.3 N/cm, average 0.55 N/cm.

### Example 9

Raw material: Chitosan fiber: linear density 2.0 dtex, fiber length 50 mm. The fiber contains 1% by weight of surfactant (Tween 20). The fiber's absorbency to the solution containing 8.298g/l of sodium chloride and 0.368 g/I of calcium chloride dehydrate (solution A) is over 110%. Acylated chitosan fiber: fiber linear density 2.2 dtex, fiber length 50 mm. fiber's absorbency of a solution containing 8.298g/l of sodium chloride and 0.368 g/I of calcium chloride dehydrate (solution A) is 800%. The fiber contains 1 % weight of Tween 20.

1900 g of chitosan fiber and 100 g of acylated chitosan fiber are blended and opened manually for 5 mins, then fed into a single cylinder card (Cuarnicard). The fibers are further blended into the hopper and the card, then opened and formed into a web. The web is crosslapped and needled into a nonwoven with a base weight of 180 gsm.

Cut the fabric into 10x10 cm, pack the dressing into pouches then sterilise the dressing by EtO.

The dressing has an absorbency of 7.2 g/g, a wet strength in CD direction of 6.9 N/cm, in MD direction of 4.8 N/cm, average 5.2 N/cm.

### Wet Strength test Method

The absorbency test for all samples of chitosan fiber, chemically modified cellulose fiber and all dressings followed the ISO standard ISO 13726-1: 2002 Part 1 Aspects of Absorbency.

The ISO standard described a Solution A as the test solution. The solution A is made up with 8.298g/l of sodium chloride and 0.368 g/I of calcium chloride dihydrate and distilled water.

In order to get an accurate reading for the dressing's wet strength, particular when comparing samples manufactured at various conditions, the test for the dressing's wet strength was performed in the following method:
1) Cut a 2 cm strip off a test specimen, the strip length shall be at least 7 cm. With a 10x10 cm wound dressing, it is preferably to cut the second sample at a 90 degree angle to the first sample, so that samples of both MD and CD directions can be obtained at the same time, as shown in Fig 4.
2) Fold the sample in half, and place the sample into the test solution 3 which is contained in the container 2. The test solution is Solution A as above. The height of the solution in the container shall be 2 +/- 0.5 cm.
3) Make sure that the sample's folded end is placed at the bottom of the device. Leave the sample in the device for 30 seconds.
4) Lift the sample out of the container, place the two ends of the sample which are still dry into the top and bottom clamps of the Tensile Tester. This will avoid the sample slippage during the tensile testing.
5) The distance between two jaws is 50 mm and the travel speed of the top jaw is set at 100 mm/min.
6) Record the maximum force (N) required to break the sample. It is recommended to test both strips of the same dressing (10x10 cm) at the same time period so that one with higher strength can be recorded as the CD, and the other as the MD.

The average wet strength is the average of CD and MD value.

## Claims

1. A wound dressing comprising chemically modified or unmodified chitosan fibers and chemically modified cellulose fibers.

2. The wound dressing comprising a blend of chemically modified or unmodified chitosan fibers and chemically modified cellulose fibers, preferably the two fibers are blended homogeneously.

3. The wound dressing as claimed in claim 1 or 2, wherein the unmodified chitosan fibers have an average absorbency of 100% - 500% of solution containing 8.298g sodium chloride and 0.368g calcium chloride dihydrate per liter and the chemically modified chitosan fibers and the chemically modified cellulose fibers have an average absorbency of 500% - 3000% of solution containing 8.298g sodium chloride and 0.368g calcium chloride dihydrate per liter.

4. The wound dressing as claimed in any one of claims 1 - 3, wherein the said wound dressing has an average wet strength of 0.3 - 6 N/cm.

5. The wound dressing as claimed in any one of claims 1 - 4, wherein the said wound dressing comprises 5 - 95% by weight of chemically modified or unmodified chitosan fibers, 95 - 5% by weight of chemically modified cellulose fibers, preferably 10 - 90% by weight of chemically modified or unmodified chitosan fibers, 90 - 10% by weight of chemically modified cellulose fibers.

6. The wound dressing as claimed in any one of claims 1 - 5, wherein the fiber linear density of chemically modified or unmodified chitosan fiber and chemically modified cellulose fiber is between 0.5 - 5 dtex, preferably 2 - 4 dtex.

7. The wound dressing as claimed in any one of claims 1 - 6, wherein the fiber length of chemically modified or unmodified chitosan fiber and chemically modified cellulose fiber is between 10mm to 125mm, preferably 25mm to 85mm.

8. The wound dressing as claimed in any one of claims 1 - 7, wherein the chemically modified or unmodified chitosan fibers and chemically modified cellulose fibers contain surfactant, or antimicrobial agent or their combinations.

9. The wound dressing as claimed in any one of claims 1 - 8, wherein the degree of deacetylation of chitosan fibers are above 50%, preferably above 70%.

10. The wound dressing as claimed in any one of claims 1 - 8, wherein the chemically modified chitosan fibers are carboxymethyl chitosan fibers or acylated chitosan fibers.

11. The wound dressing as claimed in any one of claims 1 - 10, wherein the chemically modified cellulose fibers are carboxymethyl cellulose fibers, preferably carboxymethyl solvent spun cellulose fibers.

12. The wound dressing as claimed in any one of claims 1 - 10, wherein the chemically modified cellulose fibers are sulfonated cellulose fibers, preferably sulfonated solvent spun cellulose fibers.

13. The wound dressing as claimed in any one of claims 1 - 12, wherein the wound dressing is a nonwoven felt, preferably a needle punched nonwoven felt, manufactured by blending of chemically modified or unmodified chitosan fibers and chemically modified cellulose fibers, followed by slitting, cutting, packaging and sterilisation processes.

14. The wound dressing as claimed in claim 13, wherein the dressing is manufactured by laminating one of the said fibers onto the fabric manufactured of the other fiber.

15. The wound dressing as claimed in claim 13, wherein the dressing is manufactured by laminating two pre-made fabrics together, the first fabric is manufactured by one of the said fibers, the second fabric by the other fiber.

16. The wound dressing as claimed in any one of claims 1 - 8, wherein the dressing is used in the management of chronic wound such as pressure ulcers, diabetic ulcers, venous stasis ulcer and other chronic wounds.
